Europäisches Patentamt

European Patent Office

Office européen des brevets

(19)

(11) Publication number: **0 041 749**

**A1**

(12) # EUROPEAN PATENT APPLICATION

(21) Application number: **81200564.3**

(22) Date of filing: **26.05.81**

(51) Int. Cl.³: **A 61 B 6/02**

(30) Priority: **09.06.80 NL 8003355**

(43) Date of publication of application:
**16.12.81 Bulletin 81/50**

(84) Designated Contracting States:
**DE FR GB NL**

(71) Applicant: **N.V. Philips' Gloeilampenfabrieken**
**Pieter Zeemanstraat 6**
**NL-5621 CT Eindhoven(NL)**

(72) Inventor: **Zonneveld, Frans Wessel**
**c/o INT. OCTROOIBUREAU B.V. Prof. Holstlaan 6**
**NL-5656 AA Eindhoven(NL)**

(74) Representative: **Scheele, Edial François et al,**
**INTERNATIONAAL OCTROOIBUREAU B.V. Prof.**
**Holstlaan 6**
**NL-5656 AA Eindhoven(NL)**

(54) **Medical radiation imaging apparatus.**

(57) A radiation imaging apparatus for forming three-dimensional image matrices includes means for displaying image information therefrom in scanograms which are also formed by means of the apparatus. In a scanogram thus supplemented, selected subsidiary regions are reproduced with a high resolution obtained from the image matrices and suitably positioned with respect to the patient.

EP 0 041 749 A1

Medical radiation imaging apparatus.

The invention relates to a radiation imaging apparatus, comprising a detector which is composed of a series of individually readable detector elements and which is rotatable about a patient table, either on its own or together with a radiation source, or which at least substantially surrounds the patient table, the patient table and at least the detector being arranged to be translatable with respect to one another.

A radiography apparatus of this kind is known as an X-ray scanner from British Patent Specification 1,558,155. When an apparatus described therein is used for forming scanograms by means of computer calculation of absorption values, any anomalies present in the body under examination are usually only vague by defined and their positions are difficult to establish.

A scanogram is to be understood to mean herein an image which is formed by moving an object and a detector with respect to one another in a longitudinal direction in an apparatus of the kind set forth with its rotation mechanism in a locked condition, and by recording for each location an edgewise shadowgraph image of a transaxial body section which will be called a profile, at a small distance from one another in a longitudinal direction. In principle, therefore, a scanogram is similar to a well-known shadowgraph image (radiographic), except that it is composed of a series of profiles. A profile is to be understood to mean herein a one-dimensional, flat shadowgraph image recorded from a fixed direction by means of a multi-element detector. An apparatus for forming scanograms and the advantages thereof are described in United States Patent Specification 3,101,407. When a sectional region of the object is measured in a plurality of source/detector positions which are angularly displaced about a rotation axis with respect to one another in order to form a scan-derived sectional image i.e. an image such as that customarily formed by an X-ray scanner, a scan-drived sectional image can be formed by the appropriate combination of measurement data thus obtained. A three-dimensional image matrix, also referred to as a scan-derived image, can be´formed from a series of scan-derived sectional images of consecutive planar sectional regions in an object. Measurements required to form a scan-derived sectional image will be· referred to hereinafter as a scan. It is to be noted that a profile can thus be made to constitute both to a scan-derived image and to a scanogram, so that it can supply data for the computation of a three-dimensional image matrix or data for a scanogram.

For example, for therapy purposes the radiologist requires a scanogram in which any anomalies are shown with adequate contrast and accurately positioned within their environment.

The invention has for its object to satisfy this requirement; to achieve this, a radiography apparatus of the kind set forth is characterized in that it comprises means for transferring image information from a three-dimensional image matrix to a scanogram.

In an apparatus in accordance with the invention, measurement data which concern subsidiary regions, for example, a region containing an anomaly in a patient under examination, and whose position is known exactly and in which the contrast is high, can be transferred from an arbitrarily formed three-dimensional matrix image to a scanogram or another arbitrarily formed general image.

Because a profile is an edge-projection of a scan-derived sectional image, areas in a scan-derived sectional image are thus reproduced in the profile as lines in the plane of the slice. In a scanogram a pattern of mutually parallel lines is thus formed, and when this is done in scanograms which are respectively formed using different projections, for example, one from front to back and another from one side to the other of a patient under examination, the anomaly can be unambiguously located. Obviously, this technique will also benefit the unambiguous location of an anomaly with respect to further structures in the body, for example, the location of an anomaly with respect to given bones or other suitably recognizable organs.

A preferred embodiment of the apparatus is formed by an X-ray scanner in which the radiation source and the detector are arranged to be rotatable together about the object under examination, an object carrier being arranged to be axially translatable in exact defined steps with respect to the source/detector combination.

In a further embodiment, the object under examination itself forms the radiation source, for example, a patient injected with a radioactive tracer, either only the detector being arranged to be rotatable with respect to the patient or the patient being surrounded almost completely by the detector.

In a further embodiment, a memory/arithmetic unit added to the apparatus comprises means for selecting profiles from scan-derived sectional images in dependence on their projection directions in order to form a scanogram from a consecutive series of such profiles. Thus, the need for recording a scanogram together with the series of scan-derived images throughout a relevant part of a body under examination, is eliminated. Structures from scan-derived images can also be represented in a scanogram synthetically formed in this manner.

Addition of stereotactic means to the apparatus enables the reproduction of the position and the direction of, for example, a surgical instrument, such as a needle, with respect to a marked target zone in a scano- gram filled with scan-derived image information according to the invention. An apparatus in accordance with the invention also enables radioactive tracer derived emission images and X-ray shadowgraph images of an object under examination to be correlated, so that data obtained by means of both methods can be presented in a single display.

Even though the invention will be described mainly with reference to an X-ray scanner for producing both a three-dimensional image matrix and a scanogram, the invention is not restricted thereto. The three-dimensional image matrix, for example, can alternatively be formed by using ultrasound, nuclear magnetic resonance or compton scattering measurements. However, an accurate location correlation must always exist between the three-dimensional image matrix and the scanogram.

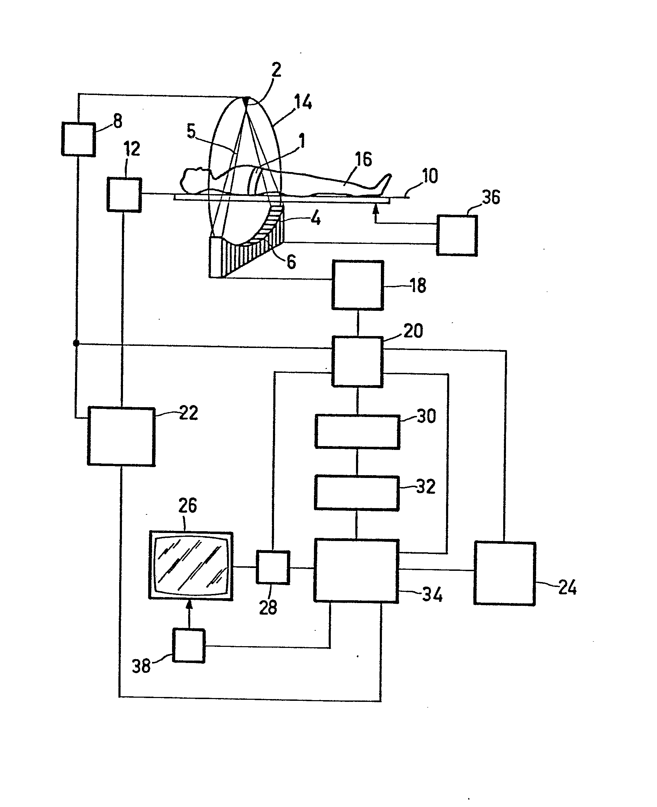

Some preferred embodiments in accordance with the invention will be described in detail hereinafter with reference to the drawing. The drawing diagrammatically shows an X-ray scanner which comprises an X-ray source 2, a detector 4 which is composed of a series of detector elements 6 which are adjacently arranged in a row and which may alternatively form a closed ring so that the detector need not be rotatable about an object 16, a high-voltage generator 8 and an object carrier 10 with a drive mechanism 12. The source 2 and the detector 4 of this embodiment are mounted on a common support 14 which enables the source and the detector to be rotated about the object 16 positioned on the object carrier. For reading the detector signals, the apparatus comprises a detector read-out unit 18 which preferably includes a signal amplifier and an analog-to-digital converter. Under the control of processed detector signals, a control device 20 controls a control mechanism 22 which controls and effects the rotation of the source/detector support, the translation of the object carrier, and the control of the high-voltage generator. The control device is furthermore connected to a memory 24 and to a signal modulator 28 which is associated with a television monitor 26. For further signal processing there are provided an arithmetic unit 30, a selection device 32 and an image computing and reconstruction device 34.

An apparatus of this kind is suitable for forming and recording a scan-derived sectional image at each of a succession of positions of the object carrier relative to the source and detector. All relevant measurement signals are processed in the detector read-out unit 18 and are applied, _via_ the control device 20 for synchronization, to the arithmetic unit 30 for further processing, or are stored in the memory 24 for processing at a later stage. A series of successive scan-derived sectional images are used to form a three-dimensional matrix of absorption values from which a sectional image which can be displayed on the monitor, can be formed in known manner. In a manner similar to the method described in United States Patent Specification 3,101,407, the apparatus can be used to form scanograms by sliding the object carrier (preferably step-wise) through the support 14 with the rotation device in a locked condition. A location indicator 36, for example, as described in German Patent Specification 26 31 809, may be useful for locating each of the profiles with respect to a reference plane. Using the arithmetic device, image points which it is desired to select from the three-dimensional matrix of absorption values can be transferred to the scanogram, after which a scanogram thus treated can be displayed on the monitor. The selection of a region of interest to be transferred from the scan-derived images to the scanograms can be indicated, for example, by means of a light pen indicator 38, in a monitor display of each scan-derived sectional image. Other forms of interactive arrangements can alternatively be used for this purpose. The arithmetic unit also enables a selection to be made of one or more projection directions, *i.e.* a selection of one or more sets of profiles, each set relating to a fixed source/object direction, from each of a series of scan-derived sectional images, and to combine the profiles relating to a set so as to form a scanogram for the selected direction.

When the detector for measuring the X-rays in an apparatus in accordance with the invention is augmented by an adjacently mounted ring of detector elements for measuring, for example, gamma rays, both X-ray images and emission images can be made using the same apparatus; the latter images are made after injection of an appropriate radio-active tracer and correlation of these images in the same display is again possible. A scanogram can thus be formed in which data obtained using the two methods is optimally combined. The possibility of exact positioning alternatively enables images recorded using different apparatus for the different types of measurement, to be combined in a single display. Preferably, the same object carrier would then be used for the various different forms of measurement. For compton scattering measurements, the source 2 in the described embodiment is, for example, a suitable high-voltage X-ray tube (approximately 350 V) to which the detector sensitivity is matched. For nuclear magnetic resonance measurements, excitation is provided by suitable electromagnetic coils with a corresponding form of detector device, and for ultrasound the source is formed by a high-frequency sound source with a detector in the form of a series of suitable transducers. Similarly, both three-dimensional image matrices and scanograms can be formed by means of a radioactive tracer introduced into the patient. In accordance with the invention, image data can then again be transferred from the three-dimensional image matrix to a scanogram or a similar type of synthetically formed general image.

CLAIMS:

1. A radiation imaging apparatus, comprising a detector which is composed of a series of individually readable detector elements and which is rotatable about a patient, either on its own or together with a radiation source, or which at least substantially surrounds the patient table, the patient table and at least the detector being arranged to be translatable with respect to one another, characterized in that there are provided means for transferring image information from a three-dimensional image matrix to a scanogram.

2. A radiation imaging apparatus as claimed in Claim 1, characterized in that it is provided with an X-ray tube which is accommodated, together with the detector, in a support which is rotatable about the patient carrier, the patient carrier being translatable with respect to the support.

3. A radiation imaging apparatus as claimed in Claim 1, characterized in that the radiation to be measured is generated in a patient under examination, the detector being arranged to measure this radiation.

4. A radiation imaging apparatus as claimed in Claim 1, 2 or 3, characterized in that, in addition to the detector for measuring radiation emitted by an X-ray source and attenuated or scattered by a patient, there is further provided a detector for measuring gamma radiation emitted by a patient under examination.

5. A radiation imaging apparatus as claimed in any of the preceding Claims, characterized in that the patient carrier includes a drive mechanism for accurately defined, step-wise displacement of the patient carrier in a direction transverse to the planes of slices to be made.

6.        A radiation imaging apparatus as claimed in any
of the preceding Claims, characterized in that it comprises an arithmetic unit for selecting on the base of
image orientation, profiles from scan-derived images and
for composing a scanogram therefrom.

7.        A radiation imaging apparatus as claimed in
any of the preceding Claims, characterized in that for a
simultaneous display of scanograms relating to different
projection directions, a plurality of television monitors
are provided.

8.        A radiation imaging apparatus as claimed in
Claim 1, 5, 6 or 7, characterized in that the radiation
source is formed by a high-frequency sound source, the
detector being formed by a series of corresponding detector transducers.

9.        A radiation imaging apparatus as claimed in
Claim 1, 5, 6 or 7, characterized in that it is provided
with a radiation source formed by an electromagnetic coil
system for generating a magnetic alternating field in the
MHz range, the detector being adapted to discriminate
local differences in the radiation absorption in a
patient.

10.        A radiation imaging apparatus as claimed in
Claim 1, 2, 5, 6 or 7, characterized in that it is provided with a high voltage X-ray tube, with a collimator
for selecting a pencil like radiation beam detector being
arranged to detect   strong radiation occurring in a body
to be examined.

0041749

European Patent
Office

EUROPEAN SEARCH REPORT

Application number

EP 81 20 0564

| Category | Citation of document with indication, where appropriate, of relevant passages | Relevant to claim |
|---|---|---|
| A | FR - A - 2 345 983 (SIEMENS A.G.)<br><br>* page 2, line 1 - page 5, line 34 and figures 1-2 * | 1,2,5, 10 |
| D | & DE - A - 2 613 809 | |
| D | US - A - 3 101 407 (J.D. SHIPMAN Jr./U.S.A. SECRETARY OF THE NAVY)<br><br>* column 1, lines 35-50; column 2, line 1 - column 3, line 48; column 4, lines 41-58 and figures 1-3 * | 1,2,10 |
| X | US - A - 4 075 700 (A.G. BLAY/EMI LTD.)<br><br>* abstract; column 1, lines 35-47; column 2, line 52 - column 3, line 13; column 3, lines 22-31 and the figure * | 1,2,6, 10 |
| | US - A - 4 179 100 (D. SASHIN et al./ UNIVERSITY OF PITTSBURGH)<br><br>* abstract; column 8, lines 11-40; column 12, lines 10-45; column 14, lines 49-60; column 15, line 62 - column 16, line 32 and figures 3a,3b,12a,12b,17, 18 and 23 * | 1-5,10 |
| | DE - A - 2 655 661 (SIEMENS A.G.)<br><br>* page 4, lines 11-28; page 5, line 10 - page 6, line 17 and figure 1 * | 1,2,5, 10 |
| | FR - A - 2 352 296 (N.V. PHILIPS' GLOEILAMPENFABRIEKEN) | 1,2,10 |

**DOCUMENTS CONSIDERED TO BE RELEVANT**

**CLASSIFICATION OF THE APPLICATION (Int. Cl.³)**

A 61 B 6/02

**TECHNICAL FIELDS SEARCHED (Int. Cl.³)**

A 61 B 6/02

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

./.

The present search report has been drawn up for all claims

| Place of search | Date of completion of the search | Examiner |
|---|---|---|
| The Hague | 17-09-1981 | RIEB |

EPO Form 1503.1 06.78

| | | |
|---|---|---|
| European Patent Office | **EUROPEAN SEARCH REPORT** | EP Application number |

| **DOCUMENTS CONSIDERED TO BE RELEVANT** | | | | CLASSIFICATION OF THE APPLICATION (Int. Cl.³) |
|---|---|---|---|---|
| Category | Citation of document with indication, where appropriate, of relevant passages | | Relevant to claim | |
| | * page 6, line 2 - page 8, line 18 and figure 1 * | | | |
| | --- | | | |
| | US - A - 4 196 352 (W.H. BERNINGER et al.) | | 1,2,10 | |
| | * abstract; column 5, line 15 - column 6, line 24 and figures 4,5 * | | | |
| | --- | | | |
| | DE - A - 1 941 433 (ELECTRIC & MUSICAL INDUSTRIES LTD.) | | 1,2,10 | |
| | | | | **TECHNICAL FIELDS SEARCHED (Int. Cl.³)** |
| | * page 3, line 1 - page 4, line 11; page 7, line 1 - page 8, line 13; page 27, lines 14-23 and figures 1-3 * | | | |
| | --- | | | |
| | US - A - 4 086 492 (J.A. LODGE et al./EMI LTD.) | | 1,2,6, 10 | |
| | * abstract; column 4, line 36 - column 5, line 31 and figure 4 * | | | |
| | --- | | | |
| | FR - A - 2 435 939 (COMPAGNIE GENERALE DE RADIOLOGIE) | | 1,2,6, 8,10 | |
| | * page 1, line 34 - page 3, line 16 * | | | |
| | --- | | | |
| E | EP - A - 0 022 722 (THOMSON-CSF) | | 1,2,6, 10 | |
| | * abstract; page 3, line 17 - page 4, line 12; page 6, line 30 - page 9, line 15; page 11, line 20 - page 13, line 18 and figures 1-5 * | | | |
| | --------- | | | |